# EUROPÄISCHE PATENTANMELDUNG

(11) **EP 4 755 425 A2**
(43) Veröffentlichungstag der Anmeldung: **10.06.2026**
(21) Anmeldenummer: 25209726.6
(22) Anmeldetag: 20.10.2025
(51) Int. Cl.: A61M 11/00, A61M 15/08, B05B 11/00

(54) **FLUIDABGABEVORRICHTUNG UND MONTAGEVERFAHREN HIERFÜR**

(30) Priorität: 06.12.2024 DE 202024107072 U
(71) Anmelder: Aero Pump GmbH, 65239 Hochheim/Main (DE)
(72) Erfinder: Rother, Sebastian, 55131 Mainz (DE); Marszalek, Milena Angelika, 55139 Mainz (DE); De Tomaso, Elena, 65462 Ginsheim (DE)
(74) Vertreter: Keil & Schaafhausen Patentanwälte PartGmbB

(57) **Zusammenfassung**

Die Erfindung betrifft eine Fluidabgabevorrichtung mit einem Pumpsystem und einem Reservoir (7) sowie ein Montageverfahren hierfür. Das Pumpsystem weist wenigstens eine Auslassöffnung (9) und eine Hülse (6) auf, die relativ zu dem Reservoir (7) bewegbar ist, um Fluid aus dem Reservoir (7) durch die Auslassöffnung (9) auszubringen. Das Reservoir (7) weist eine Außenwand, in der die Hülse (6) abdichtend geführt ist, und einen sich in Richtung der Hülse (6) erstreckenden Dorn (17) auf. Die Hülse (6) weist einen ersten Abschnitt mit einem Kanal (14) und einen zweiten Abschnitt mit einer an die Außenkontur des Dorns (17) angepassten Aufnahme auf, wobei zwischen dem Dorn (17) und der Aufnahme wenigstens ein Fluid-kanal (19, 20) ausgebildet ist.

## Beschreibung

Die Erfindung betrifft eine Fluidabgabevorrichtung mit einem Pumpsystem und einem Reservoir. Das Pumpsystem kann hierbei wenigstens eine Auslassöffnung und eine Hülse aufweisen, die relativ zu dem Reservoir bewegbar ist, um Fluid aus dem Reservoir durch die Auslassöffnung auszubringen. Weiter betrifft die Erfindung ein Verfahren zur Montage einer eine Fluidabgabevorrichtung.

Derartige Fluidabgabevorrichtungen sind häufig handbetätigte Pumpen, insbesondere Sprüh- oder Tropfpumpen, beispielsweise zur nasalen, inhalativen, buckalen, otologischen, topischen oder ophthalmologischen Verabreichung von pharmazeutischen oder kosmetischen Flüssigkeiten. Während solche Fluidabgabevorrichtungen meist für die Abgabe mehrerer Dosen des Fluids bestimmt sind, existieren auch Einwegdosierer für medizinische oder kosmetische Anwendungen, bei denen nur eine einzige Dosis des Fluids abgegeben werden soll. Beispiele hierfür sind in DE 10 2014 003 622 B4 oder EP 3 727 534 B1 beschrieben.

Es hat sich herausgestellt, dass bekannte Systeme teilweise komplexe Montageabläufe erfordern, um das Fluid mikrobiologisch dicht abzufüllen. Zudem ist das Restvolumen des Fluids, das nach Benutzung in bekannten Einwegdosierern bauartbedingt ungenutzt verbleibt teilweise relativ hoch, was gerade bei teuren Medikamenten unbefriedigend ist. In einigen Fällen wird auch der Schutz vor unbeabsichtigter Benutzung als verbesserungswürdig empfunden.

Aufgabe der vorliegenden Erfindung ist es daher, eine Fluidabgabevorrichtung sowie ein Montageverfahren hierfür anzugeben, die die oben genannten Nachteile bekannter Systeme vermeiden.

Erfindungsgemäß wird diese Aufgabe mit einer Fluidabgabevorrichtung mit den Merkmalen des Anspruchs 1 und einem Verfahren nach Anspruch 14 gelöst.

In einer erfindungsgemäßen Fluidabgabevorrichtung mit einem Pumpsystem und einem Reservoir kann das Pumpsystem wenigstens eine Auslassöffnung und eine Hülse aufweisen, die relativ zu dem Reservoir bewegbar ist, um Fluid aus dem Reservoir durch die Auslassöffnung auszubringen. Die Hülse kann dabei in das Reservoir eintauchen. Zur Minimierung des nach der einmaligen Benutzung in der Fluidabgabevorrichtung verbleibenden Rests des Fluids sind die Hülse und das Reservoir vorzugsweise aneinander angepasst. Wenn das Reservoir eine beispielsweise etwa zylindrische Außenwand, in der die Hülse abdichtend geführt ist, und einen sich vom Boden des Reservoirs aus in Richtung der Hülse erstreckenden Dorn aufweist, kann die Hülse beispielsweise einen ersten Abschnitt mit einem Kanal und einen zweiten Abschnitt mit einer an die Außenkontur des Dorns angepassten, z.B. abschnittsweise zylindrischen, Aufnahme aufweisen, wobei zwischen dem Dorn und der Aufnahme wenigstens ein Fluidkanal ausgebildet ist. Mit anderen Worten kann die Kontur der Aufnahme so an den Dorn des Reservoirs angepasst sein, dass diese aneinander anliegen und das Totvolumen somit auf das Volumen des Fluidkanals beschränkt ist. Hierdurch wird das bauartbedingt im Reservoir verbleibende Totvolumen minimiert. Der Fluidkanal dient zudem dazu, eventuelle Blockaden in der Pumpe aufgrund der engen Passungen zu vermeiden. Eine solche erfindungsgemäße Fluidabgabevorrichtung hat zudem den Vorteil, dass sie in beliebiger Orientierung im Raum verwendbar ist, weil die Hülse wie ein Kolben einer Spritze Fluid aus dem Reservoir ausbringt.

Um das bauartbedingt im Reservoir verbleibende Totvolumen zu minimieren, können die Hülse und der Dorn derart aneinander angepasst sein, dass im Wesentlichen kein Spalt zwischen der Hülse und dem Dorn vorhanden ist und diese abgesehen von dem Fluidkanal passend aneinander anliegen. Es kann jedoch auch ein kleiner Spalt zwischen Dorn und Hülse vorgesehen sein, was aufgrund von Herstellungstoleranzen vorteilhaft sein kann. Die Erfindung umfasst daher auch Ausführungsformen, bei denen ein kleiner Abstandsbereich zwischen Hülse und Dorn vorhanden ist. Dieser ist jedoch bevorzugt möglichst klein.

Nach einer bevorzugten Ausführungsform können der Dorn und die Aufnahme jeweils einen kegelförmigen Abschnitt aufweisen. Insbesondere kann die zum Pumpensystem weisende Spitze des Dorns sich verjüngend ausgebildet sein, beispielsweise als Kegelspitze oder als Kugelspitze, wobei die Aufnahme eine kongruente Innenkontur als Übergang zu dem Kanal hat, dessen Innendurchmesser vorzugsweise kleiner als der des zylindrischen Teils der Aufnahme ist.

Der Fluidkanal kann dabei entweder durch wenigstens eine Nut in dem Dorn gebildet sein und/oder durch wenigstens eine Nut in der Aufnahme. Die Nut verläuft dabei vorzugsweise in axialer Richtung, also parallel zur Mittelachse der Fluidabgabevorrichtung. Dies minimiert das Volumen des Fluidkanals und damit das Totvolumen der Fluidabgabevorrichtung.

Unabhängig davon lässt sich das Totvolumen auch dadurch weiter reduzieren, dass der Kanal der Hülse einen kleineren Innendurchmesser als die Aufnahme aufweist und/oder kürzer als die Aufnahme ist.

Das Pumpsystem der Fluidabgabevorrichtung kann weiter eine Ventilanordnung und/oder einen Zylinder aufweisen, der mit dem Kanal der Hülse strömungsverbunden ist. Die Ventilanordnung kann dabei durch ein Verschlusselement gebildet werden, das gegen die Kraft einer Feder zwischen einer eine Fluidöffnung verschließenden Stellung und einer die Fluidöffnung freigebenden Stellung in einem Hohlraum des Zylinders verschiebbar geführt ist. Dies schließt Ausgestaltungen mit ein, bei denen die Feder einstückig mit dem Verschlusselement ausgebildet ist und/oder das Verschlusselement selbst zumindest teilweise elastisch verformbar ist. Zusätzlich kann die Fluidabgabevorrichtung, insbesondere das Pumpsystem, weiter ein Kopfteil zur Aufnahme des Zylinders aufweisen, wobei in dem Kopfteil die wenigstens eine Auslassöffnung vorgesehen ist. In dem Zylinder und/oder dem Kopfteil kann ein Fluidkanal ausgebildet sein, der die Fluidöffnung mit der Auslassöffnung verbindet. Insbesondere kann dieser Fluidkanal durch eine Nut in der Außenfläche des Zylinders und/oder eine Nut in der Innenfläche des Kopfteils gebildet werden.

Das Reservoir kann in dem Kopfteil verschiebbar geführt sein, insbesondere für eine definierte Wegstrecke, die einem Ausbringhub entspricht. Der maximale Hub kann beispielsweise durch einen Anschlag der Hülse am Boden des Reservoirs begrenzt sein. Hierbei kann das Reservoir in dem Kopfteil durch eine Verrastung verliersicher gehalten sein. Insbesondere sind das Reservoir und das Kopfteil mittels hinterschnittener Bereiche derart mit einander verbunden, dass eine zerstörungsfreie Demontage der Fluidabgabevorrichtung möglichst vermieden werden kann.

Um eine unbeabsichtigte Betätigung der Fluidabgabevorrichtung zu vermeiden, kann beispielsweise das Reservoir ein abnehmbares Betätigungsschutzelement aufweisen. So kann das Betätigungsschutzelement als eine das Reservoir bereichsweise Kappe ausgebildet ist, die einen Anschlag aufweist, der derart ausgebildet ist, dass er eine Bewegung des Reservoirs in Richtung zu dem Pumpsystem verhindert oder zumindest begrenzt.

Die Fluidabgabevorrichtung kann zusätzlich eine optionale Schutzkappe aufweisen, die die Kontamination des Kopfteils verhindern soll. Weiter kann das Kopfteil optional einen radial vorstehenden Flansch aufweisen und/oder an dem Kopfteil kann ein radial vorstehender flanschartiger Fingerflügel vorgesehen sein, um die Bedienung der Fluidabgabevorrichtung zu erleichtern.

Nach einem unabhängigen Erfindungsgedanken weist ein Verfahren zur Montage einer Fluidabgabevorrichtung, insbesondere einer Fluidabgabevorrichtung der oben beschriebenen Art, folgende Schritte auf: Bereitstellen eines Reservoirs, das teilweise in einem abnehmbaren Betätigungsschutzelement aufgenommen ist, und eines Pumpsystems; Befüllen des Reservoirs mit einem Fluid, insbesondere mit einem flüssigen Wirkstoff; Aufsetzen und Verrasten des Pumpsystems auf dem Reservoir, wodurch das Betätigungsschutzelement und das Pumpsystem derart aneinander anliegen, dass eine Bewegung des Reservoirs in Richtung zu dem Pumpsystem verhindert wird. Es wird dabei besonders bevorzugt, wenn das Pumpsystem nach einem Baukastensystem gefertigt wird, bei dem Einzelteile ineinandergesteckt und mit Formschlüssen oder Presspassungen miteinander verbunden werden. So kann das Pumpsystem montiert werden, indem eine Feder und ein Verschlusselement in einen Zylinder eingesetzt werden und indem der Zylinder und eine Hülse in ein Kopfteil eingesetzt werden. Dies erlaubt eine Montage aller Bauteile nur durch axiale Bewegung parallel zur Mittelachse der Fluidabgabevorrichtung.

Die Erfindung wird im Folgenden anhand bevorzugter Ausführungsbeispiele in Verbindung mit den Zeichnungen beschrieben. Hierin zeigen schematisch:
- Figur 1: in Schnittansicht eine Fluidabgabevorrichtung nach einer ersten Ausführungsform der Erfindung vor der Benutzung;
- Figur 2: in Schnittansicht die Fluidabgabevorrichtung nach Figur 1 nach der Benutzung;
- Figur 3: in vergrößerter Schnittansicht ein Detail der Fluidabgabevorrichtung nach Figur 1 nach der Benutzung;
- Figur 4: vergrößert einen Schnitt entlang der Linie IV-IV in Figur 3;
- Figur 5: in vergrößerter Schnittansicht ein Detail der Fluidabgabevorrichtung nach einer zweiten Ausführungsform der Erfindung nach der Benutzung;
- Figur 6: vergrößert einen Schnitt entlang der Linie VI-VI in Figur 5; und
- Figur 7: in Schnittansicht eine Fluidabgabevorrichtung nach einer dritten Ausführungsform der Erfindung vor der Benutzung.

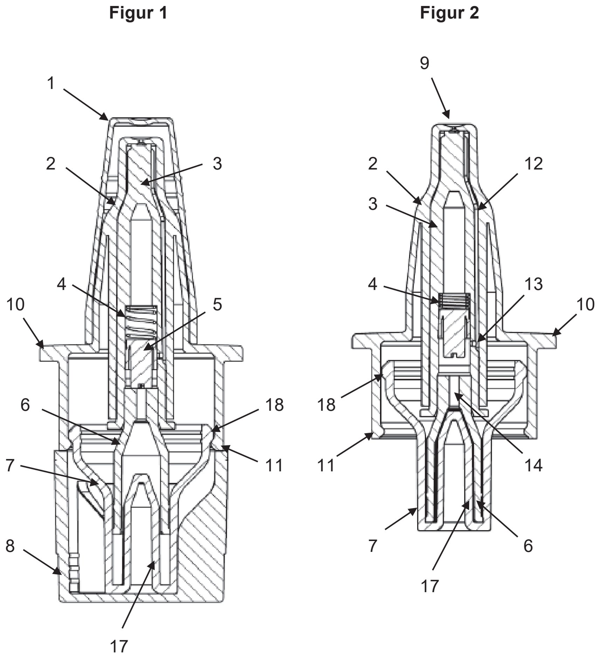

Die in Figur 1 in einem Zustand vor dem erstmaligen Gebrauch dargestellte Fluidabgabevorrichtung weist im Wesentlichen eine Schutzkappe 1, ein Kopfteil 2 (Basisteil), einen Zylinder 3 (Liner), eine Feder 4, ein als Kolben ausgebildetes Verschlusselement 5, eine Hülse 6, ein Reservoir 7 und ein Betätigungsschutzelement 8 auf. Zusätzlich kann ein nicht dargestellter Fingerflügel vorgesehen sein.

Die abnehmbare Schutzkappe 1 umschließt das Kopfteil 2 und wird vor der Benutzung der Fluidabgabevorrichtung abgenommen. Sie verhindert im dargestellten Zustand die Kontamination des Kopfteils 2.

Das Kopfteil 2 ist im Wesentlichen als eine Hülse ausgebildet, die einen Hohlraum zur Aufnahme des Zylinders 3 umschließt. An dem in der Figur oberen Ende weist das Kopfteil 2 eine Auslassöffnung 9 auf. Das Kopfteil 2 weist in der dargestellten Ausführungsform eine Kontur für das Einbringen des Kopfes in die Nase auf. Zusammen mit dem Liner bzw. Zylinder 3 bildet das Kopfteil 2 in dem dargestellten Beispiel eine Verwirbelungskammer, um das Fluid zu vernebeln. Wenn die Fluidabgabevorrichtung nicht als Nasenpumpe bzw. nicht als Spraypumpe ausgebildet ist, kann die Kontur abweichend ausgebildet sein. An dem Kopfteil 2 ist ein radial abstehender Flansch 10 als eine Fingerauflage ausgebildet. Zusätzlich kann sich auf diesem Flansch 10 ein noch weiter radial vorstehender Fingerflügel (nicht dargestellt) abstützen. Der Fingerflügel soll die Auflagefläche für die Finger verbreitern und kann mit Griffrillen das Abrutschen verhindern. Dies soll die Bedienung erleichtern. In der Figur unterhalb des Flansches 10 erstreckt sich ein z.B. zylindrischer Abschnitt mit einem nach innen gerichteten Rastwulst 11. Diese untere Kante des Kopfteils 2 bildet auch eine Anschlagfläche für das Betätigungsschutzelement 8.

Der Zylinder 3 ist in den Hohlraum des Kopfteils 2 eingesetzt und weist in dem dargestellten Beispiel ebenfalls eine Verjüngung im in der Figur oberen Bereich auf. Der Zylinder 3 leitet das Fluid über einen außenliegenden Fluidkanal 12, der mit dem Kopfteil 2 geschlossen wird, in die Verwirbelungskammer, welches zuletzt über die Auslassöffnung 9 ausgebracht wird. Der Fluidkanal 12 kann beispielsweise durch eine etwa 0,2 mm bis 1,0 mm, insbesondere etwa 0,4 mm, tiefe Nut in der Außenfläche des Zylinders gebildet werden. Alternativ oder zusätzlich kann ein solcher Fluidkanal auch durch eine entsprechende Vertiefung in der Innenfläche des Kopfteils 2 gebildet werden. Über eine seitliche Öffnung 13 ist der Fluidkanal 12 mit einem Hohlraum im Inneren des Zylinders 3 strömungsverbunden. Dieser Hohlraum weist einen Absatz auf, an dem sich die Feder 4 abstützt.

Die Feder 4 ist zusammen mit dem ein Verschlusselement 5 bildenden Kolben in den Hohlraum des Zylinders eingesetzt, so dass der Kolben gegen die Kraft der Feder 4 in der Figur nach oben verschoben werden kann. Die Feder 4 drückt den Kolben in die in Figur 1 gezeigte Position, in der der Kolben die Öffnung 13 abdichten kann. Dies ermöglicht einen Druckaufbau und ein anschließendes Verschließen der Pumpkammer nach der Benutzung. Der Kolben (Verschlusselement 5) wird u.a. dafür verwendet, um die Pumpe abzudichten. Insbesondere wird das Austreten und die Kontamination des Fluids ohne Betätigung verhindert. In dem dargestellten Beispiel weist der Kolben eine umlaufende Dichtlippe auf, mit der er gegenüber der Innenwand des Zylinders 3 abdichtet.

Die Hülse 6 weist einen ersten, zylindrisch ausgebildeten Abschnitt (oben in den Figuren) auf, in dem ein Kanal 14 ausgebildet ist. Dieser erste Abschnitt ist dichtend in den Hohlraum des Zylinders 3 eingepresst. Ein zweiter, in den Figuren unterer Abschnitt der Hülse 6 weist einen größeren Außendurchmesser als der erste Abschnitt auf und ist ebenfalls bereichsweise zylindrisch ausgebildet. Der zweite Abschnitt ist im Übergang zum ersten Abschnitt kegelförmig ausgebildet. Die Hülse 6 ist abgedichtet in dem Reservoir 7 geführt. Dafür sind eine Dichtlippe 15 und ein Führungsring 16 auf der Außenfläche der Hülse 6 vorhanden. Die Innenkontur des zweiten Abschnitts der Hülse 6 ist an die Außenkontur eines Dorns 17 des Reservoirs 7 genau angepasst.

Das Reservoir 7 dient als Behältnis für das Fluid. Es weist einen Bereich mit zylindrischer Außenwand (unterer Bereich in den Figuren) und einen Bereich mit sich aufweitendem Außendurchmesser mit einem Rastwulst 18 auf. Der Dorn 17 ragt vom Boden des Reservoirs 7 in Richtung zu der Hülse 6 auf. Damit wird zwischen der Außenwand und dem Dorn 17 ein in Figur 1 mit dem Fluid gefüllter Ringraum gebildet, in dem der zweite Abschnitt der Hülse 6 geführt ist. Die Spitze des Dorns 17 ist in dem dargestellten Beispiel an die Innenkontur der Hülse 6 angepasst kegelförmig ausgebildet.

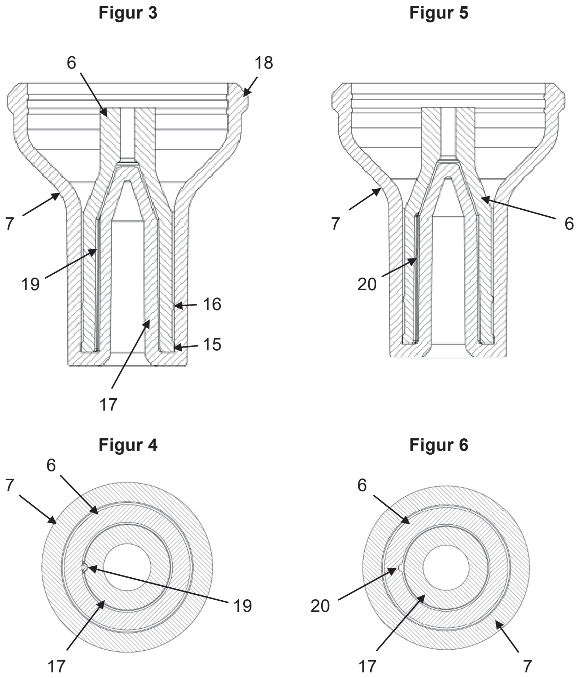

Wie aus den Figuren 3 und 4 ersichtlich ist, ist in der ersten Ausführungsform ein Fluidkanal 19 als eine Nut in der Außenfläche des Dorns 17 ausgebildet. Im Gegensatz dazu ist in der in den Figuren 5 und 6 gezeigten zweiten Ausführungsform ein Fluidkanal 20 als eine Nut in der Innenfläche des zweiten Abschnitts der Hülse 6 ausgebildet. In beiden Fällen ist diese Nut nur sehr klein, beispielsweise nur 0,2 mm bis 1,0 mm, insbesondere weniger als 0,3 mm, tief, ausgebildet, um das Totvolumen zu minimieren.

Das abnehmbare Betätigungsschutzelement 8 soll das ungewollte Betätigen der Pumpe verhindern. Dieses liegt mit einer in den Figuren oberen Kante an der unteren Kante des Kopfteils 2 an. Wenn das Betätigungsschutzelement 8 wie in Figur 1 gezeigt vorhanden ist, umgreift es das Reservoir 7, so dass dieses nicht relativ zu der Hülse 6 bewegbar ist. Erst nach dem Entfernen der Betätigungsschutzelements 8 kann die Pumpe betätigt werden.

Die Befüllung der Fluidabgabevorrichtung erfolgt, indem zunächst das Reservoir 7 in das Betätigungsschutzelement 8 eingesetzt und der Ringraum zwischen Außenwand und Dorn 17 mit einer bestimmten Menge des Produkts gefüllt wird. Die übrigen Komponenten, nämlich das Kopfteil 2, der Zylinder 3, die Feder 4, das Verschlusselement (Kolben) 5 und die Hülse 6, werden zu einer Pumpenbaugruppe vormontiert, insbesondere indem diese ineinandergesteckt und, soweit sie nicht zueinander beweglich sein sollen, mit Formschlüssen oder Presspassungen miteinander verbunden.

Diese Pumpenbaugruppe wird dann auf die Baugruppe aus Reservoir 7 und Betätigungsschutzelement 8 aufgeprellt. Der Anschlag des Betätigungsschutzelements 8 mit dem Kopfteil 2 sichert die korrekte Position des Reservoirs 7. Rastwulst 11 und Rastwulst 18 hintergreifen sich hierbei und verhindern, ein Herausziehen des Reservoirs 7 aus dem Kopfteil 2, wenn das Betätigungsschutzelement 8 entfernt wird. Die Funktion des Betätigungsschutzelements 8 besteht darin, das Reservoir 7 während der Füllungsphase mittels Stegen sicher zu halten und das Reservoir 7 korrekt in den Kopfteil 2 zu leiten. Der Anschlag des Betätigungsschutzelements 8 zu dem Kopfteil 2 ist so ausgelegt, dass das Reservoir 7 ohne das Risiko in den Kopfteil 2 geprellt wird, dass das Produkt vorzeitig nach außen fließt. Zur Betätigung der Pumpe muss das Betätigungsschutzelement 8 dann abgezogen werden, aber das Reservoir 7, das durch den Hinterschnitt der Rastwülste 11 und 18 im Kopfteil 2 gehalten wird, muss in seiner Position bleiben. D.h. die Demontagekraft des Betätigungsschutzelements 8 ist vorzugsweise geringer sein als die Demontagekraft des Reservoirs 7 vom Kopfteil 2.

Nachfolgend wird die für beide Ausführungsformen identische Funktionsweise der Pumpe erläutert. Der Anwender muss zum Betätigen der Pumpe zunächst die Schutzkappe 1 und das Betätigungsschutzelement 8 entfernen.

Anschließend wird die Pumpe beispielsweise in die Nase gebracht und betätigt, indem das Reservoir 7 gegen die Hülse 6 in Richtung des Kopfteils 2, gedrückt wird. Die Hülse 6 ist dabei in dem Reservoir 7 abgedichtet geführt und das Volumen in dem Reservoir 7 wird reduziert. Somit muss sich die darin befindliche Flüssigkeit durch den Fluidkanal 19 bzw. 20 und den Kanal 14 in Richtung des Kolbens 5 bewegen. Dieser verschiebt sich bei Erreichen eines definierten Drucks gegen die Kraft der Feder 4 und gibt hierdurch die Öffnung 13 frei, so dass die Flüssigkeit durch den Fluidkanal 12 in die Verwirbelungskammer eingeführt wird. Ein Vergleich der Position des Kolbens des Verschlusselements 5 in den Figuren 1 und 2 zeigt die Verschiebung des Kolbens sowie das Komprimieren der Feder 4, so dass die Öffnung 13 in Figur 1 von dem Kolben geschlossen ist und in Figur 2 freigegeben ist. Nach der c verlässt das Fluid zerstäubt durch die Auslassöffnung 9 die Pumpe.

Nach einem vollständigen Hub des Reservoirs 7 relativ zu der Hülse 6 stoßen diese im Bereich des Bodens des Reservoirs 7 wie in Figur 2 gezeigt aneinander an, wodurch das Reservoir 7 soweit möglich, d.h. bis auf das bauartbedingt in dem Fluidkanal 19 bzw. 20 verbleibende Totvolumen, entleert ist. Auch in dem Kanal 14, der Öffnung 13 und dem Fluidkanal 12 verbleibt ein geringer Rest des Fluids, der nicht ausgebracht werden kann.

Die gezeigte Gestaltung des Reservoirs 7 und der Hülse 6 mit nur einem Fluidkanal 19 bzw. 20 und den kegelförmigen Konturen am Dorn 17 und der Hülse 6 bewirkt, dass beispielsweise das Totvolumen, das nach einem Hub des Reservoirs 7 wie oben beschrieben in dem Raum zwischen Reservoir 7 und Hülse 6, insbesondere in dem Fluidkanal 19 bzw. 20, verbleibt, weniger als 10%, insbesondere weniger als 5%, beispielsweise etwa 4%, des auszubringenden Volumens beträgt.

Zudem erlaubt die in den Figuren gezeigte Gestaltung des Reservoirs 7 mit einem im Boden ausgebildeten Dorn 17 und einem sich aufweitenden Bereich mit dem Rastwulst 18 eine Herstellung des Reservoirs als Kunststoff-Spritzgussteil. Vorzugsweise sind auch die übrigen Bauteile der erfindungsgemäßen Fluidabgabevorrichtung, ggf. mit Ausnahme der Feder 4, Kunststoff-Spritzgussteile.

Die Erfindung ist beispielhaft in den Figuren für einen Nasenspray als Einwegdosierer, d.h. mit einer Monodose-Pumpe, gezeigt, mit der der Inhalt des Reservoirs 7 bei einem einzigen Hub ausgebracht wird. Die Vorteile des geringen Totvolumens lassen sich aber in gleicher Weise auch für andere Pumpentypen, beispielsweise für Tropfer-Pumpen, bei denen das Fluid nicht zerstäubt wird, oder für inhalative, bukkale, otologische, topische oder ophthalmologische Verabreichungen nutzen. In diesen Fällen kann insbesondere das Kopfteil 2 eine von der Darstellung der Figuren abweichende Form haben.

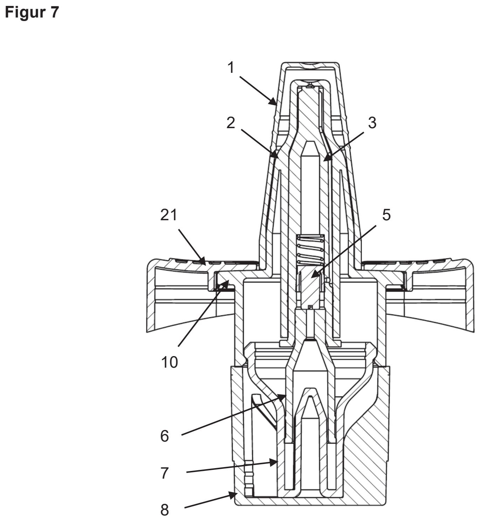

Figur 7 zeigt eine Fluidabgabevorrichtung ähnlich der Ansicht von Figur 1, jedoch mit einem zusätzlichen Fingerflügel 21, der an dem Flansch 10 anliegend die Betätigung erleichtert.

Nach einem unabhängigen Aspekt der vorliegenden Erfindung weist ein Verfahren zur Montage einer Fluidabgabevorrichtung, insbesondere einer Fluidabgabevorrichtung der oben beschriebenen Art, folgende Schritte auf:
- Bereitstellen eines Reservoirs 7, das teilweise in einem abnehmbaren Betätigungsschutzelement 8 aufgenommen ist, und eines Pumpsystems,
- Befüllen des Reservoirs 7 mit einem Fluid, insbesondere mit einem flüssigen Wirkstoff,
- Aufsetzen und Verrasten des Pumpsystems auf dem Reservoir 7, wodurch das Betätigungsschutzelement 8 und das Pumpsystem derart aneinander anliegen, dass eine Bewegung des Reservoirs 7 in Richtung zu dem Pumpsystem verhindert wird.

Dieses Verfahren ist optional dadurch gekennzeichnet, dass das Pumpsystem montiert wird, indem eine Feder 4 und ein Verschlusselement 5 in einen Zylinder 3 eingesetzt werden und indem der Zylinder 3 und eine Hülse 6 in ein Kopfteil 2 eingesetzt werden.

### Bezugszeichen

- 1: Schutzkappe
- 2: Kopfteil
- 3: Zylinder
- 4: Feder
- 5: Verschlusselement (Kolben)
- 6: Hülse
- 7: Reservoir
- 8: Betätigungsschutzelement
- 9: Auslassöffnung
- 10: Flansch
- 11: Rastwulst
- 12: Fluidkanal
- 13: Öffnung
- 14: Kanal
- 15: Dichtlippe
- 16: Führungsring
- 17: Dorn
- 18: Rastwulst
- 19: Fluidkanal
- 20: Fluidkanal
- 21: Fingerflügel

## Patentansprüche

1. Fluidabgabevorrichtung mit einem Pumpsystem und einem Reservoir (7), wobei das Pumpsystem wenigstens eine Auslassöffnung (9) und eine Hülse (6) aufweist, die relativ zu dem Reservoir (7) bewegbar ist, um Fluid aus dem Reservoir (7) durch die Auslassöffnung (9) auszubringen, **dadurch gekennzeichnet, dass** das Reservoir (7) eine Außenwand, in der die Hülse (6) abdichtend geführt ist, und einen sich in Richtung der Hülse (6) erstreckenden Dorn (17) aufweist, und dass die Hülse (6) einen ersten Abschnitt mit einem Kanal (14) und einen zweiten Abschnitt mit einer an die Außenkontur des Dorns (17) angepassten Aufnahme aufweist, wobei zwischen dem Dorn (17) und der Aufnahme wenigstens ein Fluidkanal (19, 20) ausgebildet ist.

2. Fluidabgabevorrichtung nach Anspruch 1, **dadurch gekennzeichnet, dass** der Dorn (17) und die Aufnahme jeweils einen kegelförmigen Abschnitt aufweisen.

3. Fluidabgabevorrichtung nach Anspruch 1 oder 2, **dadurch gekennzeichnet, dass** der Dorn (17) und die Aufnahme jeweils einen zylindrischen Abschnitt aufweisen.

4. Fluidabgabevorrichtung nach Anspruch 1 bis 3, **dadurch gekennzeichnet, dass** der Fluidkanal (19) durch wenigstens eine Nut in dem Dorn (17) gebildet ist.

5. Fluidabgabevorrichtung nach Anspruch 1 bis 3, **dadurch gekennzeichnet, dass** der Fluidkanal (20) durch wenigstens eine Nut in der Aufnahme der Hülse (6) gebildet ist.

6. Fluidabgabevorrichtung nach einem der vorhergehenden Ansprüche, **dadurch gekennzeichnet, dass** der Kanal (14) der Hülse (6) einen kleineren Innendurchmesser als die Aufnahme aufweist und/oder kürzer als die Aufnahme ist.

7. Fluidabgabevorrichtung nach einem der vorhergehenden Ansprüche, **dadurch gekennzeichnet, dass** das Pumpsystem einen Zylinder (3), in dem ein Verschlusselement (5) gegen die Kraft einer Feder (4) zwischen einer eine Öffnung (13) verschließenden Stellung und einer die Öffnung (13) freigebenden Stellung verschiebbar geführt ist, wobei der Zylinder (3) mit dem Kanal (14) der Hülse (6) strömungsverbunden ist.

8. Fluidabgabevorrichtung nach Anspruch 7, **dadurch gekennzeichnet, dass** das Pumpsystem weiter ein Kopfteil (2) zur Aufnahme des Zylinders (3) aufweist, in dem die Auslassöffnung (9) vorgesehen ist, wobei in dem Zylinder (3) und/oder dem Kopfteil (2) ein Fluidkanal (12) ausgebildet ist, der die Öffnung (13) mit der Auslassöffnung (9) verbindet.

9. Fluidabgabevorrichtung nach Anspruch 8, **dadurch gekennzeichnet, dass** das Reservoir (7) in dem Kopfteil (2) verschiebbar geführt ist.

10. Fluidabgabevorrichtung nach Anspruch 8 oder 9, **dadurch gekennzeichnet, dass** das Reservoir (7) in dem Kopfteil (2) verrastet ist.

11. Fluidabgabevorrichtung nach einem der vorhergehenden Ansprüche, **dadurch gekennzeichnet, dass** das Reservoir (7) ein abnehmbares Betätigungsschutzelement (8) aufweist.

12. Fluidabgabevorrichtung nach Anspruch 11, **dadurch gekennzeichnet, dass** das Betätigungsschutzelement (8) als eine das Reservoir (7) bereichsweise Kappe ausgebildet ist, die einen Anschlag aufweist, der derart ausgebildet ist, dass er eine Bewegung des Reservoirs (7) in Richtung zu dem Pumpsystem verhindert oder begrenzt.

13. Fluidabgabevorrichtung nach einem der Ansprüche 8 bis 12, **dadurch gekennzeichnet, dass** das Kopfteil (2) einen radial vorstehenden Flansch (10) aufweist, und/oder dass an dem Kopfteil (2) ein radial vorstehender flanschartiger Fingerflügel (21) vorgesehen ist.

14. Verfahren zur Montage einer Fluidabgabevorrichtung, insbesondere einer Fluidabgabevorrichtung nach einem der vorhergehenden Ansprüche, wobei das Verfahren folgende Schritte aufweist:
• Bereitstellen eines Reservoirs (7), das teilweise in einem abnehmbaren Betätigungsschutzelement (8) aufgenommen ist, und eines Pumpsystems,
• Befüllen des Reservoirs (7) mit einem Fluid, insbesondere mit einem flüssigen Wirkstoff,
• Aufsetzen und Verrasten des Pumpsystems auf dem Reservoir (7), wodurch das Betätigungsschutzelement (8) und das Pumpsystem derart aneinander anliegen, dass eine Bewegung des Reservoirs (7) in Richtung zu dem Pumpsystem verhindert wird.

15. Verfahren nach Anspruch 14, **dadurch gekennzeichnet, dass** das Pumpsystem montiert wird, indem eine Feder (4) und ein Verschlusselement (5) in einen Zylinder (3) eingesetzt werden und indem der Zylinder (3) und eine Hülse (6) in ein Kopfteil (2) eingesetzt werden.
